# EUROPÄISCHE PATENTANMELDUNG

(11) **EP 1 166 724 A1**
(43) Veröffentlichungstag der Anmeldung: **02.01.2002**
(21) Anmeldenummer: 00810543.9
(22) Anmeldetag: 21.06.2000
(51) Int. Cl.: A61F 2/30, A61F 2/36, A61F 2/46, A61F 2/34

(54) **Gelenkteil einer Interimsprothese sowie Interimsprothese**

(71) Anmelder: Sulzer Orthopedics Ltd., 6340 Baar (CH)
(72) Erfinder: Die Erfindernennung liegt noch nicht vor
(74) Vertreter: Sulzer Management AG

(57) **Zusammenfassung**

Es wird ein Gelenkteil einer Interimsprothese für ein Kugelgelenk vorgeschlagen mit einem Kugelkopf (2) zum Zusammenwirken mit einer künstlichen oder natürlichen Gelenkpfanne sowie mit einem dünnen, länglichen Stützkörper (3), welcher fest mit dem Kugelkopf (2) verbunden ist, wobei der Kugelkopf (2) aus einem Knochenzement hergestellt ist.

## Beschreibung

Die Erfindung betrifft ein Gelenkteil einer Interimsprothese für ein Kugelgelenk sowie eine Interimsprothese mit einem solchen Gelenkteil.

Es ist bekannt, dass es bei implantierten Gelenkendoprothesen zu Infektionen des Knochengewebes kommen kann, insbesondere in den Bereichen, in denen die Verankerungsteile der Prothese fixiert sind. In solchen Fällen kann es notwendig sein, die Prothese oder Teile davon im Rahmen einer Revisionsoperation zu explantieren und die entzündeten Knochenbereiche medikamentös zu behandeln. Hierzu ist es beispielsweise bekannt, in einer einzigen Revisionsoperation die Primärprothese zu entfernen und die definitive Revisionsprothese zu implantieren, wobei die Revisionsprothese oder der zu ihrer Verankerung verwendete Knochenzement ein Antibiotikum enthält, um die Infektion zu behandeln. Diese auch als einzeitige Revision bekannte Methode ist jedoch mit dem relativ grossen Risiko behaftet, dass die Infektion nicht vollständig beseitigt wird und daher eine erneute Operation notwendig ist.

Bei den sogenannten zweizeitigen Revisionen umfasst die Revisionsoperation zwei separate Operationen. In der ersten Operation wird die ursprüngliche Prothese entfernt. Die endzündeten Knochenbereiche werden üblicherweise sowohl systemisch, das heisst oral oder intravenös, als auch unmittelbar mit einem Antibiotikum behandelt. Dazu wird in die Kavität, die an das entzündete Knochengewebe angrenzt, also beispielsweise der Markhohlraum, welcher den Verankerungsschaft der Prothese aufnimmt oder die Kavität für die Gelenkpfanne, ein temporäres Implantat aus Knochenzement wie Polymethylmetacrylat (PMMA) eingebracht, dem ein Antibiotikum beigemischt ist. Das Antibiotikum wandert aus dem Knochenzement in das Knochengewebe, wo es therapeutisch wirksam wird. Wenn die Entzündung abgeklungen ist, was typischerweise einige Wochen bis Monate dauern kann, wird der antibiotikumhaltige Knochenzement in der zweiten Operation der Revision entfernt und die Prothese bzw. die Prothesenteile werden implantiert. Das Einfüllen von Knochenzement hat den Vorteil, dass ein nachteiliges Verkürzen von Bändern, Sehnen und Muskeln, damit einhergehende Beinverkürzung und ein Schrumpfen der Kavität oder ein Einwachsen des Knochengewebes in die Kavität verhindert wird. Der antibiotikumhaltige Knochenzement hat also neben seiner therapeutischen Funktion auch die Funktion eines Platzhalters für die Prothese, welcher-den Raum für die Prothesenteile freihält.

In der Regel ist es notwendig, während der gesamten Behandlungsdauer mittels des antibiotischen Knochenzements das betroffene Gelenk vollständig zu immobilisieren, beispielsweise durch externe Fixateure oder andere geeignete Massnahmen. Dies ist insbesondere im Falle der Kugelgelenke (Hüftgelenke, Schultergelenke) relativ schwierig und aufwendig. Zudem kommt es durch die vollständige Immobilisierung über mehrere Wochen zu nachteiligem Muskelschwund.

Wird beispielsweise wegen einer Entzündung des Knochengewebes im Femur ein Prothesenschaft explantiert und die Markraumkavität zur Behandlung der Endzündung mit einem antibiotischen Knochenzement gefüllt, so ist die Gelenkfunktion vollständig ausser Kraft gesetzt und die Verbindung zwischen dem Oberschenkel und dem Körper ist nur noch über Weichteile, wie Bänder, Muskeln oder Sehnen realisiert. Selbst bei geringfügigen Bewegungen des Patient besteht dann bereits die erhebliche Gefahr, dass es zu Verletzungen, beispielsweise dem Abklemmen von Blutgefässen kommt.

Es ist daher eine Aufgabe der Erfindung, eine Vorrichtung für die Behandlung entzündeten Knochengewebes im Bereich von Kugelgelenken mit antibiotischem Knochenzement bereitzustellen, welche die erwähnten Probleme löst. Die Vorrichtung soll es also insbesondere ermöglichen, die temporäre antibiotische Behandlung ohne eine vollständige Immobilisierung des betroffenen Gelenks durchzuführen. Ferner soll die Vorrichtung möglichst einfach sowie kostengünstig sein und auch intraoperativ eine möglichst gute und einfache Anpassung an die individuellen Gegebenheiten des Patienten ermöglichen.

Diese Aufgabe wird durch ein Gelenkteil einer Interimsprothese bzw. durch eine Interimsprothese gelöst, wie es bzw. sie durch die Merkmale im jeweiligen unabhängigen Patentanspruch gekennzeichnet ist. Vorteilhafte Massnahmen und bevorzugte Ausgestaltungen der Erfindung ergeben sich aus den Merkmalen der abhängigen Ansprüche.

Erfindungsgemäss wird also ein Gelenkteil einer Interimsprothese für ein Kugelgelenk vorgeschlagen, mit einem Kugelkopf zum Zusammenwirken mit einer künstlichen oder natürlichen Gelenkpfanne sowie mit einem dünnen, länglichen Stützkörper, welcher fest mit dem Kugelkopf verbunden ist, wobei der Kugelkopf aus einem Knochenzement hergestellt ist.

Zur Behandlung von entzündetem Knochengewebe wird an den Stützkörper des vorgefertigten Gelenkteils intraoperativ ein Schaft aus Knochenzement mit einem darin enthaltenen Antibiotikum angeformt, wobei der Schaft von seiner Form an die vorhandene Markraumhöhle im Knochen angepasst ist. Der Stützkörper, welcher den Kugelkopf trägt, wird dann mit dem angeformten Schaft in analoger Weise wie dies für einen Schaft einer dauerhaften Prothese üblich ist, in die Markraumhöhle eingesetzt und fixiert, bis der Knochenzement ausgehärtet ist. Es ist aber auch möglich, die Markraumhöhle mit antibiotischem Knochenzement zu füllen, dann den Stützkörper in den noch plastischen Knochenzement hineinzudrücken, und den Knochenzement aushärten zu lassen. Der fest mit dem Stützkörper verbundene Kugelkopf wird in die natürliche oder eine künstliche Gelenkpfanne eingesetzt, sodass die Gelenkfunktion auch während der Behandlung des entzündeten Knochengewebes erhalten bleibt. Zudem ist gewährleistet, dass die Markraumhöhle nicht schrumpft, sodass das Gelenkteil zusammen mit dem angeformten Schaft aus antibiotischem Knochenzement auch effizient als Platzhalter für das endgültige Prothesenteil fungiert.

Das einzementierte Gelenkteil bildet zusammen mit der Gelenkpfanne eine Interimsprothese, welche nur für die Dauer der Behandlung des entzündeten Knochengewebes implantiert ist. Anschliessend wird das Gelenkteil explantiert und ein endgültiges oder dauerhaftes Prothesenteil implantiert. Die Interimsprothese macht somit eine vollständige Immobilisierung des betroffenen Gelenks unnötig und gewährleistet ferner, zumindest in eingeschränktem Masse, eine Belastbarkeit des Gelenks.

Das erfindungsgemässe Gelenkteil lässt sich sehr einfach an die individuellen Gegenheiten des Patienten anpassen, weil der Schaft aus antibiotikumhaltigen Knochenzement erst intraoperativ an den dünnen Stützkörper angeformt wird, bzw. der dünne Stützkörper in den noch plastischen Knochenzement eingedrückt wird. Somit eignet sich das Gelenkteil für praktisch alle Geometrien der Markraumkavität.

Da der Kugelkopf aus Knochenzement besteht, kann er besonders einfach und kostengünstig hergestellt werden. Zudem resultiert der Vorteil, dass dem Knochenzement für den Kugelkopf ein Antibiotikum beigemischt werden kann bzw. ist, sodass der Kugelkopf vollständig oder mindestens an seiner Artikulationsfläche mit dem Antibiotikum versetzt ist, das nach der Implantation an das angrenzende Knochengewebe abgegeben wird. Falls der Kugelkopf also ein Antibiotikum enthält, können auch Entzündungen im Bereich der Gelenkpfanne therapiert werden.

Vorzugsweise besteht der Stützkörper aus Metall oder Kunststoff, , um die mechanische Belastbarkeit der Interimsprothese zu erhöhen.

In einer bevorzugten Ausgestaltung umfasst der Stützkörper mehrere Einkerbungen. Zum einen verbessern diese die Verankerung des Stützkörpers im Knochenzement und zum anderen erleichtern sie das intraoperative Anpassen der Länge des Stützkörpers an die Anatomie des Patienten bzw. an die Länge der vorhandenen Markraumhöhle, weil an diesen Einkerbungen eine einfache Kürzung des Stützkörpers möglich ist.

Eine weitere vorteilhafte Massnahme besteht darin, den Kugelkopf mit einer abnehmbaren Ummantelung, insbesondere aus einem Silikongummi, zu umgeben. Diese Ummantelung schützt die Artikulationsfläche des Kugelkopfs vor Verunreinigungen oder Beschädigungen, beispielsweise während des Einsetzens des Stützkörpers in den Knochenzement. Vor dem Einsetzen des Kugelkopfs in die Gelenkpfanne wird die Ummantelung entfernt.

Der Kugelkopf kann zum Zusammenwirken mit einer künstlichen Gelenkpfanne ausgestaltet sein und weist dann einen Durchmesser von höchstens 38 mm - 50 mm auf, weil dies üblicherweise der maximale Innendurchmesser künstlicher Hüftgelenkpfannen ist.

Es ist aber auch möglich, dass der Kugelkopf so bemessen ist, dass er mit einer natürlichen Gelenkpfanne zusammenwirken kann, beispielsweise mit der noch vorhandenen natürlichen Gelenkpfanne oder mit der Kavität, die nach der Explantation einer künstlichen Gelenkpfanne vorhanden ist. Der Kugelkopf wirkt dann unmittelbar mit natürlichem Knochengewebe zusammen. Der Durchmesser des Kugelkopfs beträgt dann typischerweise bis zu etwa 60 mm für Hüftanwendungen und bis zu etwa 40 mm für Schulteranwendungen.

Eine weitere vorteilhafte Massnahme besteht darin, an dem Gelenkteil Mittel zum Erleichtern des Ausbauens vorzusehen. Dies kann beispielsweise eine am Stützkörper vorgesehene Verlängerung sein, die so ausgebildet ist, dass an ihr ein Ausschlaginstrument befestigt werden kann.

Die erfindungsgemässe Interimsprothese für ein Kugelgelenk umfasst ein erfindungsgemässes Gelenkteil sowie eine Gelenkpfanne zum Zusammenwirken mit dem Gelenkteil, wobei die Gelenkpfanne aus einem Knochenzement besteht. Diese Interimsprothese ist für die vorübergende Implantation gedacht. Die aus Knochenzement hergestellte Gelenkpfanne dient als Platzhalter (Spacer), welcher es verhindert, dass die Kavität für die später zu implantierende endgültige Gelenkpfanne schrumpft oder das Knochengewebe in diese Kavität hineinwächst. Es kann vorteilhaft sein, die Gelenkpfanne der Interimsprothese mittels Schrauben oder ähnlicher Mittel im Knochen zu fixieren.

Ferner ist es möglich, die Gelenkpfanne der Interimsprothese aus einem antibiotikumhaltigen Knochenzement herzustellen, sodass die Gelenkpfanne ein Antibiotikum enthält. Dies hat den Vorteil, dass mit einer solchen Interimsprothese zusätzlich entzündetes Knochengewebe im Bereich der Gelenkpfanne behandelt werden kann.

Das erfindungsgemässe Gelenkteil bzw. die erfindungsgemässe Interimsprothese können in sinngemäss gleicher Weise sowohl für das Hüftgelenk als auch für das Schultergelenk ausgestaltet sein.

Im Folgenden wird die Erfindung anhand von Ausführungsbeispielen und anhand der Zeichnung näher erläutert. In der Zeichnung zeigen schematisch und teilweise im Schnitt:
- Fig. 1:: eine Längsschnittdarstellung eines Ausführungsbeispiels eines erfindungsgemässen Gelenkteils im implantierten Zustand,
- Fig. 2:: eine Vorrichtung zum Herstellen des Gelenkteils,
- Fig. 3:: ein Ausführungsbeispiel einer Gelenkpfanne einer erfindungsgemässen Interimsprothese im Schnitt, und
- Fig. 4:: ein Vorrichtung zum Herstellen der Gelenkpfanne aus Fig. 3.

Im Folgenden wird die Erfindung anhand eines Ausführungsbeispiels für Hüftanwendungen erläutert. Es versteht sich aber, dass die Erfindung in sinngemäss gleicher Weise auch für Anwendungen im Schulterbereich geeignet ist, wobei dann die Abmessungen der einzelnen Teile entsprechend kleiner sind.

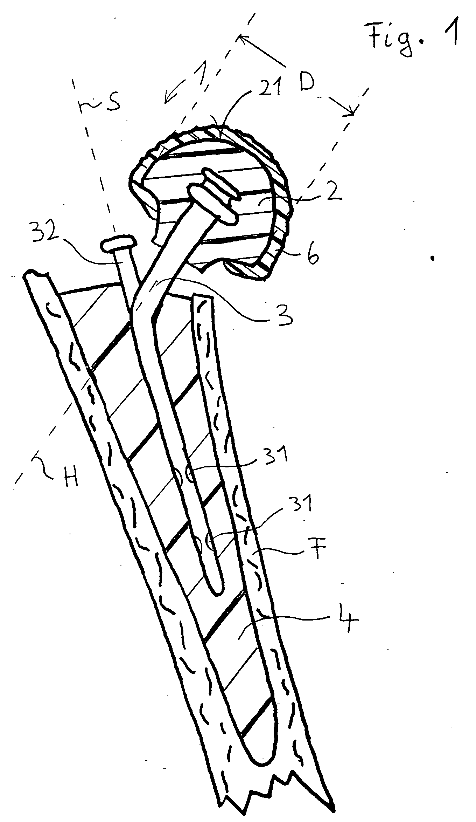

Fig. 1 zeigt in einer Längsschnittdarstellung ein Ausführungsbeispiel eines erfindungsgemässen Gelenkteils einer Interimsprothese für ein Kugelgelenk. Das Gelenkteil ist gesamthaft mit dem Bezugszeichen 1 versehen und in einem Femur F implantiert dargestellt. Mit dem Begriff "Interimsprothese" ist gemeint, dass diese im Unterschied zur der endgültigen oder definitiven Prothese nur vorübergehend für einige Wochen oder wenige Monate implantiert ist und dann wieder entfernt wird. Die Interimsprothese dient der Behandlung von entzündetem Knochengewebe, beispielsweise im Rahmen einer Revisionsoperation, und fungiert gleichzeitig als Platzhalter (Spacer), welcher ein Schrumpfen der Weichteile (Bänder, Sehnen, Muskel) und der Kavität im Knochen bzw. ein Einwachsen von Knochengewebe in die Kavität verhindert, sodass die definitive Prothese nach Entfernen der Interimsprothese ohne Probleme in die vorhandene Kavität eingesetzt werden kann.

Das Gelenkteil 1 umfasst einen Kugelkopf 2 zum Zusammenwirken mit einer künstlichen oder einer natürlichen Hüftgelenkpfanne, sowie einen dünnen, länglichen Stützkörper 3, welcher fest mit dem Kugelkopf 2 verbunden ist.

Mit dem Begriff "natürliche Hüftgelenkpfanne" sind auch solche Kavitäten gemeint, die nach der Explantation einer künstlichen Hüftgelenkpfanne vorhanden sind.

Bezüglich seines prinzipiellen Verlaufs ist der Stützkörper 3 im wesentlichen gleich ausgebildet wie der Hüftschaft einer definitiven Prothese, das heisst der Stützkörper weist in seinem proximalen Bereich einen Knick bzw. eine Biegung auf, sodass er eine Schaftlängsachse S und eine Halslängsachse H hat, die sich unter dem sogenannten CCD-Winkel (Centrum-Collum-Diaphyse-Winkel) schneiden. Der CCD-Winkel liegt im Bereich von 120° bis 150° und insbesondere beträgt er 130°-135°. Im Vergleich zu dem Hüftschaft einer definitiven, heute üblichen Prothese ist der Stützkörper 3 bezüglich seiner transversalen Ausdehnung senkrecht zur Längserstreckung wesentlich dünner, das heisst die transversale Ausdehnung ist auch deutlich kleiner als der Innendurchmesser der Markraumhöhle im Femur F. Heute übliche Hüftschäfte definitiver Prothesen haben unter Berücksichtigung der Anatomie eine transversale Ausdehnung von 8 mm bis 24 mm. Der Stützkörper 3 hat dann beispielsweise eine transversale Abmessung von etwa 50% - 60 % der entsprechenden Abmessung des Hüftschafts, also zwischen 4 mm und 15 mm, wobei die kleineren Werte, insbesondere 5 mm bis 8 mm, für die transversale Abmessung des Stützkörpers 3 bevorzugt sind. Der Stützkörper 3 ist jedenfalls so dünn, dass er im implantierten Zustand keinen unmittelbaren Kontakt mit dem die Markraumhöhle begrenzenden Knochengewebe hat. In diesem Sinne ist die Bezeichnung "dünner Stützkörper" zu verstehen.

Um der mechanische Belastbarkeit der Interimsprothese zu verbesseren, besteht der Stützkörper 3 vorzugsweise aus einem Metall, beispielsweise aus rostfreiem Stahl oder Titan, oder aus einem harten Kunststoff, beispielsweise PMMA, PEEK (Polyetheretherketon) oder POM (Polyoxymethylen).

Der Teil des Stützkörpers 3, der sich in Richtung der Schaftlängsachse S erstreckt, ist mit mehreren Einkerbungen 31 versehen. Diese Einkerbungen 31 dienen zum einen dazu, dass der Stützkörper 3 intraoperativ in einfacher Weise gekürzt werden kann, um ihn an die Länge der Markraumhöhle anzupassen. Auf Höhe jeder Einkerbung 31 kann der Stützkörper 3 abgebrochen oder geschnitten werden, um ihn so an die Anatomie des Patienten anzupassen. Zum anderen verbessern die Einkerbungen 31 die Verankerung des Stützkorpers 3 in dem ihn umgebenden Knochenzement 4.

Optional kann der Stützkörper 3 noch mit Mitteln zum Erleichtern des Ausbauens versehen sein. Bei dem hier beschriebenen Ausführungsbeispiel (siehe Fig. 1) ist am Stützkörper 3 eine Verlängerung 32 vorgesehen, die sich in Richtung der Schaftlängsachse S erstreckt und deren proximales Ende so ausgestaltet ist, dass es mit einem Ausschlaginstrument verbunden werden kann. Durch diese Massnahme wird die Explantation des Gelenkteils 1 erleichtert.

Der Kugelkopf 2 ist erfindungsgemäss aus einem Knochenzement, beispielsweise Polymethylmetacrylat (PMMA), hergestellt und bei dem hier beschriebenen Ausführungsbeispiel direkt an den Stützkörper 3 angeformt. Die äussere Form des Kugelkopfs 2, welche die Artikulationsfläche 21 bildet, entspricht der Kugelform einer üblichen Gelenkkugel einer Hüftgelenkprothese.

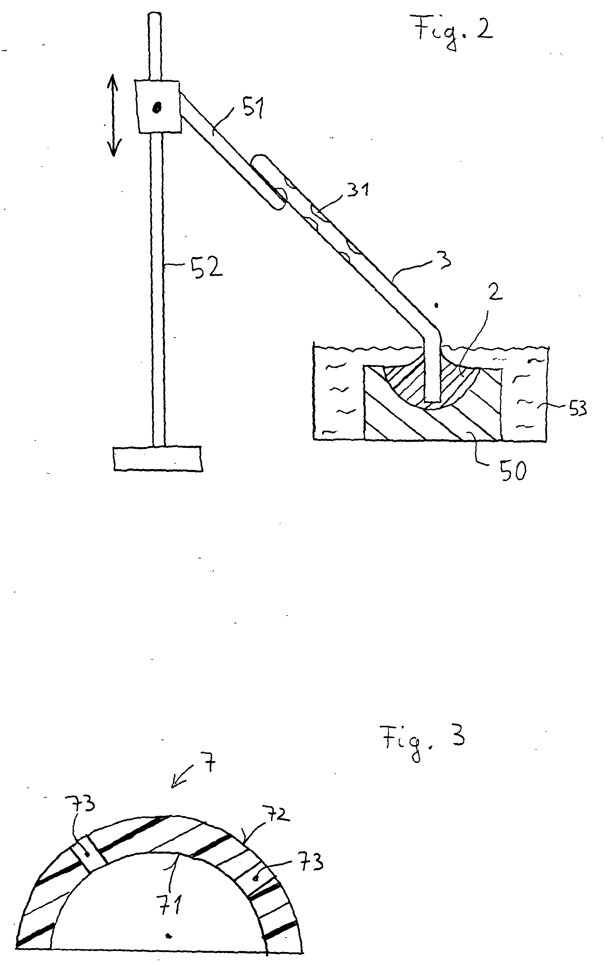

Zur Herstellung des Gelenkteils 1 wird beispielsweise wie folgt vorgegangen (siehe die schematische Darstellung in Fig. 2). Eine sterilisierbare Giessform 50, die beispielsweise aus Polyoxymethylen (POM) oder einem anderen geeigneten Material besteht, wird mit dem Knochenzement gefüllt. Die Giessform 50 weist eine kugelkopfförmige Kavität auf, deren konkave Begrenzungsfläche die Formgebungsfläche für den Kugelkopf 2 bildet und deren Innendurchmesser dem gewünschten Durchmesser D (Fig. 1) des Kugelkopfs 2 des herzustellenden Gelenkteils 1 entspricht. Der vorgefertigte Stützkörper 3 wird mit seinem proximalen Ende zentral in den noch nicht harten Knochenzement in der Giessform 50 eingebracht und mit seinem distalen Endbereich an einer Fixierung 51 befestigt. Die Fixierung 51 ist höhenverstellbar (angedeutet durch den Doppelpfeil in Fig. 2) und arretierbar an einem Halter 52 gelagert. Durch Verschieben der Fixierung 51 in Richtung des Doppelpfeils wird der Stützkörper 3 relativ zum Kugelkopf 2 justiert. Dann wird die Fixierung 51 am Halter 52 arretiert und der Knochenzement in der Giessform 50 durch Polymerisation aushärten gelassen. Um während der Polymerisation eine Blasenbildung im Knochenzement zu vermeiden, befindet sich die Giessform 50 in einer Wasserkühlung 53. Nach Aushärtung des Knochenzements wird das Gelenkstück 1 aus der Giessform 50 entfernt und gegebenenfalls noch nachbearbeitet (was jedoch in der Regel nicht mehr notwendig ist).

Vorzugsweise wird der Kugelkopf 2 mit einer abnehmbaren Ummantelung 6 (siehe Fig. 1) versehen, um ihn bzw. die Artikulationsfläche 21 beim Einbau des Gelenkteils vor Verschmutzungen, beispielsweise durch Knochenzement, und vor Verletzungen zu schützen. Die Ummantelung 6 ist vorzugsweise als elastischer Überzug, beispielsweise aus einem Silikongummi, ausgestaltet, damit sie in einfacher Weise vom Kugelkopf 2 abnehmbar bzw. auf diesen aufbringbar ist.

Die für den Operationssaal vorgefertigten Gelenkteile 1 werden mit verschiedenen Durchmessern D des Kugelkopfs 2 hergestellt. Falls der Kugelkopf 2 zum Zusammenwirken mit einer künstlichen Hüftgelenkpfanne ausgestaltet ist, werden die verschiedenen Durchmesser D entsprechend der für den Innendurchmesser der künstlichen Hüftgelenkpfannen üblichen Abstufung gewählt. Das heisst, in der Regel steht im Operationssaal ein Satz von Gelenkteilen 1 zur Verfügung, die verschiedene Durchmesser D des Kugelkopfs 2 aufweisen. Diese Durchmesser D entsprechen jeweils den Durchmessern des Kugelkopfs, die für die definitiven Prothesen gebräuchlich sind. Für das Zusammenwirken mit einer künstlichen Hüftgelenkpfanne beträgt der Durchmesser des Kugelkopfs 2 des Gelenkteils 1 höchstens 50 mm für Hüftanwendungen.

Der Kugelkopf 2 des Gelenkteils 1 kann aber auch so bemessen werden, dass er mit einer natürlichen Gelenkpfanne zusammenwirkt bzw. mit der Kavität im Beckenknochen, die nach der Explantation einer künstlichen Gelenkpfanne vorhanden istl. Im Falle des Zusammenwirkens mit einer natürlichen Gelenkpfanne beträgt der Durchmesser D des Kugelkopf typischerweise bis zu 60 mm für Hüftanwendungen und bis zu 40 mm für Schulteranwendungen.

Natürlich ist es auch möglich, das Gelenkteil 1 intraoperativ herzustellen. Dazu wird im Rahmen der Operation zunächst die vorhandene Prothese explantiert. Mit einer Testpfanne wird dann der korrekte Durchmesser für den Kugelkopf 2 am Patienten ermittelt. Anschliessend wird das Gelenkteil 1 wie vorangehend beschrieben hergestellt. Dazu wird aus einem noch ungebogenen Metallstück geeigneter Länge, beispielsweise einer Knochenplatte wie sie als Knochenverschraubung verwendet wird, zunächst der Stützkörper 3 gebogen. Dieser wird dann mit seinem einen Ende in den noch weichen Knochenzement in der Giessform 50 eingebracht und der Knochenzement aushärten gelassen. Es versteht sich, dass im Operationssaal mehrere Giessformen 50 vorhanden sind, die unterschiedliche Innendurchmesser aufweisen, sodass der Kugelkopf 2 mit dem für den jeweiligen Patienten ermittelten Durchmesser herstellbar ist.

Im Folgenden wird nun am Beispiel einer Revisionsoperation ein mögliches Verfahren beschrieben, wie das vorgefertigte oder intraoperativ hergestellte Gelenkteil 1 in den Femur (oder ananlog in den Humerus) eingesetzt wird, bzw. wie die Interimsprothese implantiert wird, wobei wiederum auf das Beispiel einer Hüftanwendung Bezug genommen wird.

Zunächst wird der alte Hüftschaft und gegebenenfalls die Gelenkpfanne der Prothese explantiert,und die Markraumhöhle im Femur F sowie das Acetabulum im Becken werden gereinigt. Dann wird ein Gelenkteil 1 ausgewählt, dessen Kugelkopf 2 einen Durchmesser D hat, welcher zu der natürlichen Gelenkpfanne passt. Es wird kontrolliert, ob der Stützkörper 3 zu lang für die Markraumhöhle ist, falls ja wird der Stützkörper 3 an einer der Einkerbungen 31 durch Abbrechen oder Abschneiden auf die passende Länge gekürzt.

Der mit einem Antibiotikum, beispielsweise Gentamicin, versetzte Knochenzement wird für die Implantation vorbereitet. Nun wird händisch um den Stützkörper 3 herum mit dem antibiotikumhaltigen Knochenzement 4 ein Schaft angeformt, der etwa der Form der Markraumkavität entspricht. Im noch plastischen Zustand des antibiotikumhaltigen Knochenzements wird das Gelenkteil 1 mit dem angeformten Schaft in den Femur F eingesetzt und dort justiert bzw. zentriert. Danach wird das Gelenkteil 1 fixiert, bis der Knochenzement 4 ausgehärtet ist. Bei diesen Arbeiten befindet sich die Ummantelung 6 auf dem Kugelkopf 2, sodass dieser vor Verunreinigungen, z. B. durch Knochenzement, oder Beschädigungen geschützt ist. Nun wird die Ummantelung 6 entfernt und der Kugelkopf 2 in die natürliche oder künstliche Hüftgelenkpfanne reponiert, sodass die Interimsprothese fertiggestellt ist.

Um die spätere Entfernung des Gelenkteils 1 zu vereinfachen, kann es vorteilhaft sein, den an das Gelenkteil 1 angeformten Schaft aus Knochenzement vor dem Einsetzen in die Knochenkavität mit Wasser zu benetzen.

Die Interimsprothese bleibt über einen Zeitraum von einigen Wochen bis zu beispielsweise vier Monaten implantiert und dient während dieser Zeit als Platzhalter für die definitive Prothese und als Träger bzw. Speicher für das Antibiotikum, welches aus dem Knochenzement 4 in das angrenzende, entzündete Knochengewebe übergeht und dort therapeutisch wirksam wird. Der Fortschritt der Heilung des Knochengewebes lässt sich durch Beobachtung von typischen Infektionsparametern wie Fieber, Blutwerte usw. kontrollieren.

Während dieser Behandlungsphase bleibt durch die Interimsprothese die Gelenkfunktion erhalten und zudem ist zumindest in eingeschränktem Masse eine Belastbarkeit des Gelenks gewährleistet.

Nach erfolgreicher Behandlung, das heisst nach Abklingen der Infektion des Knochengewebes wird das Gelenkstück 1 zusammen mit dem angeformten Schaft aus Knochenzement entnommen, beispielsweise indem an der Verlängerung 32 ein an sich bekanntes Ausschlaginstrument befestigt wird und das Gelenkstück 1 mit dem Knochenzement ausgeschlagen wird. Anschliessend wird in an sich bekannter Weise der definitive Prothesenschaft bzw. die definitive Prothese implantiert.

Zur Implantation des Gelenkteils 1 ist es auch möglich, die Markraumkavität im Femur F mit dem antibiotikumhaltigen Knochenzement 4 zu füllen, dann das Gelenkstück 1 in den noch plastischen Knochenzement 1 hineinzudrücken, zu justieren bzw. zu zentrieren und das Gelenkstück 1 anschliessend solange zu fixieren, bis der Knochenzement 4 ausgehärtet ist.

Ferner ist auch eine Kombination der beiden Verfahren möglich. Dazu wird an den Stützkörper 3 aus Knochenzement oder aus antibiotikumhaltigem Knochenzement ein Schaft angeformt, der dünner bzw. kleiner ist als die Markraumkavität im Femur F. Die Markraumkavität wird dann vorzugsweise unvollständig mit antibiotikumhaltigen Knochenzement 4 gefüllt und das Gelenkteil 1 mit dem dünnen, angeformten Schaft aus Knochenzement eingesetzt.

Das erfindungsgemässe Gelenkstück 1 hat den Vorteil, dass es sehr einfach und auch kostengünstig in der Herstellung ist. Da der Kugelkopf 2 des Gelenkteils 1 aus Knochenzement hergestellt ist, sind die Materialkosten gering und zudem ist die Herstellung beispielsweise im Vergleich zu einem Kugelkopf aus Metall deutlich einfacher, weil der Kugelkopf 2 beispielsweise wie vorne beschrieben mit geringem Aufwand in einer Giessform 50 herstellbar ist.

Eine weitere vorteilhafte Massnahme besteht darin, den Kugelkopf 2 des Gelenkteils 1 ganz oder zumindest teilweise, nämlich insbesondere im Bereich der Artikulationsfläche 21, aus einem antibiotikumhaltigen Knochenzement, beispielsweise PMMA mit Gentamicin, herzustellen, sodass auch der Kugelkopf 2 ein Antibiotikum enthält. Vorzugsweise ist der Kugelkopf 2 aus Knochenzement mit Antibiotikum gefertigt. Auch ein solcher Kugelkopf 2 kann in der Giessform 50 hergestellt werden. Diese Massnahme ist insbesondere bei solchen Anwendungen vorteilhaft, bei denen der Kugelkopf 2 des Gelenkteils 1 mit einer natürlichen Gelenkpfanne zusammenwirkt. Da der Kugelkopf 2 unmittelbar mit Knochengewebe in Kontakt kommt, ohne dass eine künstliche Schale dazwischen ist, kann das Antibiotikum aus dem Kugelkopf 2 in das benachbarte Knochengewebe übergehen, um dort therapeutisch wirksam zu werden. Somit lassen sich mit einer solchen Interimsprothese zusätzlich Entzündungen des Knochengewebes im Bereich der Gelenkpfanne therapieren.

Insbesondere bei grösseren Defekten des Acetabulums (bzw. der Schultergelenkpfanne) ist es vorteilhaft, wenn die Interimsprothese zusätzlich zu dem Gelenkteil 1 eine Gelenkpfanne umfasst, die aus einem Knochenzement besteht. Fig. 3 zeigt ein Ausführungsbeispiel einer solchen Gelenkpfanne 7 in einer Schnittdarstellung. Die konkave innere Begrenzungsfläche bildet die Artikulationsfläche 71, welche derart bemessen ist, dass sie mit dem Kugelkopf 2 des Gelenkteils 1 zusammenwirken kann. Die äussere Begrenzungsfläche 72 ist anatomisch angepasst. Optional kann die Gelenkpfanne 7 noch mit Bohrungen oder Löchern 73 versehen sein, durch welche Schrauben zur Fixierung der Gelenkpfanne 7 in den Knochen eingedreht werden können. Vorzugsweise enthält die Gelenkpfanne ein Antibiotikum, beispielsweise Gentamicin, welches an das benachbarte Knochengewebe abgegeben wird. Dazu wird die Gelenkpfanne 7 vorzugsweise aus einem antibiotikumhaltigen Knochenzement hergestellt.

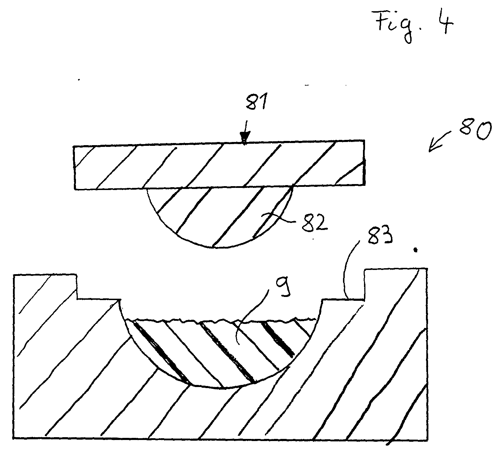

Fig. 4 zeigt in einer schematischen Darstellung eine Giessform 80 zur Herstellung einer Gelenkpfanne 7. Die Giessform 80 weist eine konkave Kavität auf, in welche der Knochenzement 9, dem vorzugsweise ein Antibiotikum beigemischt ist, eingefüllt wird. Die Giessform 80 besteht beispielsweise aus POM. Ferner ist ein Stempel 81 vorgesehen, der einen Formkörper 82 umfasst, dessen äussere Begrenzungsfläche sphärisch gekrümmt ausgestaltet ist, wobei die Krümmung durch den gewünschten Innenradius der herzustellenden Gelenkschale 7 festgelegt ist. Nachdem der Knochenzement 9 in die Kavität eingebracht ist, wird der Stempel 81 in den noch nicht gehärteten Knochenzement 9 gedrückt, bis er auf einem Absatz 83, aufliegt, welcher die seitliche Begrenzung der Kavität entlang ihres Umfangs bildet. Auf diese Weise formt der Formkörper 82 des Stempels 81 die Artikulationsfläche 71 der Gelenkpfanne 7. Nachdem der Knochenzement ausgehärtet ist, wird die Gelenkpfanne 7 entformt und gegebenenfalls noch nachbearbeitet.

Es versteht sich, dass auch die Gelenkpfanne 7 aus Knochenzement bzw. die zu ihrer Herstellung notwendigen Formen in mehreren Grössen, das heisst insbesondere mit verschiedenen Innendurchmesser bereitgestellt werden, sodass der Arzt während der Operation die passende Gelenkpfanne 7 auswählen kann.

Die Gelenkpfanne 7 der Interimsprothese dient als Platzhalter für die endgültige Hüftgelenkpfanne und vermeidet zuverlässig ein Schrumpfen der Knochenkavität während des Zeitraums bis zur Implantation der definitiven Prothese. Falls die Gelenkpfanne 7 aus antibiotikahaltigem Knochenzement hergestellt ist, dient sie gleichzeitig als Reservoir für das Antibiotikum, welches aus der Gelenkpfanne 7 in das benachbarte Knochengewebe übergeht, um dort therapeutisch wirksam zu werden.

Beim Einbau der Interimsprothese wird die Gelenkpfanne 7 in die vorbereitete Knochenkavität eingesetzt und dort mittels Knochenzement, der vorzugsweise antibiotikumhaltig ist, fixiert. Da sich dieser Knochenzement sehr gut mir dem Knochenzement verbindet, aus dem die Gelenkpfanne besteht, ist eine gute Verankerung gewährleistet. Optional kann jedoch die Gelenkpfanne 7 zusätzlich oder alternativ auch mittels Schrauben im Knochen verankert werden. Bei der Implantation der definitiven Prothese, wird die Gelenkschale 7 aus Knochenzement durch eine definitive Gelenkschale ersetzt.

## Patentansprüche

1. Gelenkteil einer Interimsprothese für ein Kugelgelenk mit einem Kugelkopf (2) zum Zusammenwirken mit einer künstlichen oder natürlichen Gelenkpfanne sowie mit einem dünnen, länglichen Stützkörper (3), welcher fest mit dem Kugelkopf (2) verbunden ist, wobei der Kugelkopf (2) aus einem Knochenzement hergestellt ist.

2. Gelenkteil nach Anspruch 1, bei welchem der Stützkörper (3) aus Metall oder Kunststoff besteht.

3. Gelenkteil nach Anspruch 1 oder 2, bei welchem der Stützkörper (3) mehrere Einkerbungen (31) umfasst.

4. Gelenkteil nach einem der vorangehenden Ansprüche, bei welchem der Kugelkopf (2) mit einer abnehmbaren Ummantelung (6), insbesondere aus einem Silikongummi, umgeben ist.

5. Gelenkteil nach einem der vorangehenden Ansprüche, bei welchem der Kugelkopf (2) aus einem Knochenzement mit Antibiotikum gefertigt ist.

6. Gelenkteil nach einem der vorangehenden Ansprüche, wobei der Kugelkopf (2) zum Zusammenwirken mit einer künstlichen Gelenkpfanne ausgestaltet ist und einen Durchmesser von höchstens 50 mm aufweist.

7. Gelenkteil nach einem der Ansprüche 1-5, wobei der Kugelkopf (2) so bemessen ist, dass er mit einer natürlichen Gelenkpfanne zusammenwirken kann.

8. Gelenkteil nach einem der vorangehenden Ansprüche, mit Mittel (32) zum Erleichtern des Ausbauens.

9. Interimsprothese für ein Kugelgelenk mit einem Gelenkteil (1) gemäss einem der vorangehenden Ansprüche sowie mit einer Gelenkpfanne (7) zum Zusammenwirken mit dem Gelenkteil (1), wobei die Gelenkpfanne (7) aus einem Knochenzement besteht.

10. Interimsprothese nach Anspruch 9, bei welchem die Gelenkpfanne (7) ein Antibiotikum enthält.
